# EUROPEAN PATENT SPECIFICATION

(11) **EP 4 108 249 B1**
(45) Date of publication and mention of the grant of the patent: **28.01.2026**
(21) Application number: 21756799.9
(22) Date of filing: 17.02.2021
(51) Int. Cl.: A61K 35/747, A61K 36/489, A61P 15/12, A61P 3/04, A23L 33/135, A23L 33/105

(54) **COMPOSITION FOR TREATING CLIMACTERIC DISORDER COMPRISING LACTOBACILLUS GASSERI BNR17**
ZUSAMMENSETZUNG ZUM BEHANDELN VON KLIMAKTERISCHEN STÖRUNGEN, DIE LACTOBACILLUS GASSERI BNR17 UMFASST
COMPOSITION POUR LE TRAITEMENT DE TROUBLE CLIMATÉRIQUE COMPRENANT LACTOBACILLUS GASSERI BNR17

(30) Priority: 17.02.2020 KR 20200019129
(43) Date of publication of application: 28.12.2022
(73) Proprietor: Acebiome Inc., Seongnam-si, Gyeonggi-do 13488 (KR)
(72) Inventor: PARK, Han-Oh, Sejong 30151 (KR); KIM, Myeong Hee, Sejong 30151 (KR); JUNG, Bong Jun, Gimpo-si Gyeonggi-do 10068 (KR); YOO, Wonbeak, Daejeon 34061 (KR)
(74) Representative: Mewburn Ellis LLP
(86) International application number: PCT/KR2021/002034
(87) International publication number: WO 2021/167350

(56) References cited:
- KR-A- 20040 038 481
- KR-A- 20080 048 976
- KR-A- 20110 004 603
- KR-A- 20110 081 202
- KR-A- 20130 002 543
- US-A1- 2013 171 253
- MUHLEISEN ALICIA L ET AL: "Menopause and the vaginal microbiome", MATURITAS, ELSEVIER, AMSTERDAM, NL, vol. 91, 1 June 2016 (2016-06-01), pages 42 - 50, XP029649867, ISSN: 0378-5122, DOI: 10.1016/J.MATURITAS.2016.05.015
- LEE SOL ET AL: "The Effect of Lactobacillus gasseri BNR17 on Postmenopausal Symptoms in Ovariectomized Rats", JOURNAL OF MICROBIOLOGY AND BIOTECHNOLOGY, vol. 31, no. 9, 28 September 2021 (2021-09-28), Korea, pages 1281 - 1287, XP093089105, ISSN: 1017-7825, DOI: 10.4014/jmb.2105.05032

## Description

### [Technical Field]

The present invention relates to a pharmaceutical composition for use in the treatment of climacteric syndrome containing *Lactobacillus gasseri* BNR17 deposited as KCTC 10902BP as an active ingredient and a functional food composition for use in the prevention or amelioration of climacteric symptoms containing *Lactobacillus gasseri* BNR17 deposited as KCTC 10902BP as an active ingredient.

### [Background Art]

Climacteric syndrome is a term that collectively refers to various symptoms caused by a rapid decrease in estrogen, which is a female hormone, following menopause in women. Recently, estrogen supplementation therapy has been used, but long-term use thereof causes side effects such as uterine bleeding, breast cancer, strokes, uterine cancer, and the like, so plant-derived *Sophora japonica* fruit extracts have been receiving attention as an alternative thereto. *Sophora japonica* fruit extracts contain abundant sophoricoside, which is a type of isoflavone, and have been clinically confirmed to be effective at alleviating climacteric symptoms in women (Journal of Pharmacy, Vol. 49, No. 4 (2005), pp. 317-322).

Meanwhile, *Lactobacillus gasseri* BNR17 derived from human breast milk is a lactic acid bacterium that exhibits strong acid resistance, bile resistance, and intestinal adhesion, so it inhabits the body and converts low-saccharide carbohydrates degraded by digestive enzymes into high-molecular-weight polysaccharide materials that are not absorbed in the body to thus discharge the same, showing a confirmed effect of prevention or treatment of diabetes (Korean Patent Application Publication No. 10-2008-0048976).

The present inventors have been searching for ways to ameliorate climacteric symptoms to improve the quality of life of women after middle age due to the increased average human lifespan expectancy, and ascertained that a *Lactobacillus gasseri* BNR17 strain is effective at alleviating various climacteric symptoms such as amelioration of osteoporosis, amelioration of involutional depression, amelioration of pain hypersensitivity, and amelioration of vaginal dryness, thus culminating in the present invention.

KR 10-2008-0048976 A (BIONEER CORP [KR]) discloses composition for Prevention and Treatment of Diabetes Mellitus with Lactobacillus gasseri BNR17. MUHLEISEN ALICIA L ET AL ("Menopause and the vaginal microbiome" MATURITAS, ELSEVIER, AMSTERDAM, NL, vol. 91, 1 June 2016 (2016-06-01), pages 42-50, XP029649867, ISSN: 0378-5122) discusses menopause and the vaginal microbiome. KR 10-2011-0081202 A (MEIJI DAIRIES CORPORATION) discloses lactic acid bacterium having high oxalic acid decomposition ability.

### [Disclosure]

The invention is as defined in the claims. This invention and other facets of the present disclosure may be more fully understood by reference to the following description.

It is an object of the present invention to provide novel use of the *Lactobacillus gasseri* strain recited in the claims.

In order to accomplish the above object, the present invention provides a pharmaceutical composition for use in the treatment of climacteric syndrome containing *Lactobacillus gasseri* BNR17 deposited as KCTC 10902BP as an active ingredient.

In addition, the present invention provides a functional food composition for use in the prevention or amelioration of climacteric symptoms containing *Lactobacillus gasseri* BNR17 deposited as KCTC 10902BP as an active ingredient.

Disclosed herein, but not part of the claimed invention, is a method of treating climacteric syndrome including administering *Lactobacillus gasseri* BNR17 to a subject requiring treatment for climacteric syndrome.

Disclosed herein, but not part of the claimed invention, is a method of preventing or ameliorating climacteric symptoms including administering *Lactobacillus gasseri* BNR17 to a subject requiring prevention or amelioration of climacteric symptoms.

Disclosed herein, but not part of the claimed invention, is the use of *Lactobacillus gasseri* BNR17 for the treatment of climacteric syndrome or the prevention or amelioration of climacteric symptoms.

Disclosed herein, but not part of the claimed invention, is the use of *Lactobacillus gasseri* BNR17 for the manufacture of a medicament for the treatment of climacteric syndrome.

Disclosed herein, but not part of the claimed invention, is the use of *Lactobacillus gasseri* BNR17 for the manufacture of a functional food for the prevention or amelioration of climacteric symptoms.

### [Description of Drawings]

FIG. 1 shows the results of analysis of changes in calcitonin, osteocalcin, and serotonin in the blood due to administration of *Lactobacillus gasseri* BNR17 according to the present invention in a climacteric animal model;
FIG. 2 shows the results of analysis of changes in deoxypyridinoline in the urine due to administration of *Lactobacillus gasseri* BNR17 according to the present invention in a climacteric animal model;
FIG. 3 shows results confirming the amelioration of vaginal cornification due to administration of *Lactobacillus gasseri* BNR17 according to the present invention in a climacteric animal model; and
FIG. 4 shows the results of measurement of changes in femoral bone density due to administration of *Lactobacillus gasseri* BNR17 according to the present invention in a climacteric animal model.

### [Mode]

Unless otherwise defined, all technical and scientific terms used herein have the same meanings as those typically understood by those skilled in the art to which the present disclosure belongs. In general, the nomenclature used herein is well known in the art and is typical.

In the present disclosure, administration of *Lactobacillus gasseri* BNR 17 derived from human breast milk to an ovariectomized climacteric animal model has confirmed the effects of amelioration of climacteric symptoms, such as suppression of increased pain sensitivity, amelioration of climacteric indicators in the blood and urine, an increased proportion of cornified vaginal epithelial cells, an increase in bone density, and amelioration of osteoporosis.

Accordingly, an aspect of the present invention pertains to a pharmaceutical composition for use in the treatment of climacteric syndrome containing *Lactobacillus gasseri* BNR17 (Accession No. KCTC 10902BP) as an active ingredient.

In the present invention, the treatment of climacteric syndrome may be selected from the group consisting of (i) amelioration of osteoporosis, (ii) alleviation of involutional depression, (iii) amelioration of pain hypersensitivity, and (iv) amelioration of vaginal dryness, but the present invention is not limited thereto.

In the present invention, the composition for use may further include a *Sophora japonica* extract, but is not limited thereto, and in order to enhance the effect thereof, it may be administered in combination with a known material having proven efficacy in treating climacteric syndrome.

In the present invention, the *Sophora japonica* extract may be a *Sophora japonica* fruit extract, but is not limited thereto.

The pharmaceutical composition for use of the present invention may be provided as a composition that may be combined with live cells, a dry strain form, a strain culture, a strain lysate, or a pharmaceutically acceptable carrier or medium therefor. Examples of the carrier or medium that is used include solvents, dispersants, coating agents, absorption enhancers, controlled release agents (i.e. sustained release agents), and one or more inert excipients (starch, polyol, granules, microfine cellulose, microcrystalline cellulose (e.g. Celphere, Celphere beads), diluents, lubricants, binders, disintegrants, etc.). As necessary, a tablet formulation of the disclosed composition may be coated through a standard aqueous or non-aqueous technique. Examples of excipients useful as pharmaceutically acceptable carriers and pharmaceutically acceptable inert carriers and additional ingredients include, but are not limited to, binders, fillers, disintegrants, lubricants, antimicrobial agents, and coating agents.

The pharmaceutical composition for use of the invention is capable of exhibiting an effect of treating climacteric syndrome, and unless otherwise stated, the term 'treating' as used herein means reversing, alleviating, inhibiting the progression of, or preventing the disease or condition to which the term is applied, or one or more symptoms of the disease or condition, and the term 'treatment' as used herein refers to the act of treating when 'treating' is defined as above.

The pharmaceutical composition for use includes an effective amount of *Lactobacillus gasseri* BNR17 and optionally a *Sophora japonica* extract, or may further include one or more pharmaceutically acceptable carriers, excipients, or diluents.

In the present disclosure, the term "effective amount" means an amount that is sufficiently large to realize the desired effect but is sufficiently small to prevent serious side effects, determined within the scope of medical judgment. The amount of the composition that is administered into the body may be appropriately adjusted in consideration of the route of administration and the subject of administration.

The composition for use of the present invention may be administered to a subject one or more times daily. A unit dosage means physically discrete units suitable for unit administration to human subjects and other mammals, each unit including a suitable pharmaceutical carrier and containing a predetermined amount of the composition for use of the present invention to thereby exhibit a therapeutic effect. The dosage unit for oral administration to an adult patient preferably contains 0.001 g or more of the composition for use of the present invention, and the oral dosage of the composition for use of the present invention is 0.001 to 10 g, preferably 0.01 to 5 g, at a time. A pharmaceutically effective amount of the composition for use of the present invention is 0.01 to 10 g/day. However, the dosage varies depending on the severity of the patient's disease and the microorganism and additional active ingredients that are used. Moreover, the total daily dosage may be divided into several doses and administered repeatedly as needed.

In addition, as used herein, "pharmaceutically acceptable" refers to a composition that is physiologically acceptable and does not normally cause allergic reactions such as gastrointestinal disorders and dizziness or similar reactions when administered to humans.

The composition for use of the present invention may be formulated using methods known in the art to provide rapid, sustained, or delayed release of the active ingredient after administration to a mammal. Formulations may be in the form of powders, granules, tablets, emulsions, syrups, aerosols, soft or hard gelatin capsules, sterile injectable solutions, or sterile powders. Moreover, the composition for use may be administered through various routes including oral, transdermal, subcutaneous, intravenous, or intramuscular routes, and the dosage of the active ingredient may be appropriately determined depending on various factors including the route of administration, age, gender, and body weight of the patient, and the severity of disease. The composition for use may be administered in combination with a known compound having an effect of preventing, ameliorating, or treating symptoms thereof.

The pharmaceutical composition for use of the present invention may be provided as an enteric coating formulation, in particular, in a unit dosage form for oral use. As used herein, "enteric coating" includes any known pharmaceutically acceptable coating that is capable of remaining in the stomach without being degraded by gastric acid and being sufficiently degraded in the small intestine so that the active ingredient is released into the small intestine. The "enteric coating" may be a coating that remains unchanged for 2 hours or more when brought into contact with artificial gastric juice such as an HCl solution at a pH of 1 at 36°C to 38°C and preferably degrades thereafter within 30 minutes in artificial intestinal juice such as a KH₂PO₄ buffer solution at a pH of 6.8.

The enteric coating may be coated in an amount of about 16 to 30 mg, preferably 16 to 20 or 25 mg per core. When the thickness of the enteric coating is 5 to 100 µm, preferably 20 to 80 µm, satisfactory results are obtained as an enteric coating. The material for the enteric coating is appropriately selected from among known polymeric materials. Suitable polymeric materials are listed in a number of known publications (L. Lachman et al., The Theory and Practice of Industrial Pharmacy, 3rd ed., 1986, pp. 365-373), and may include cellulose ester derivatives, cellulose ethers, methyl acrylate copolymers of acrylic resins, and copolymers of maleic acid and phthalic acid derivatives.

The enteric coating may be prepared using a typical enteric coating method in which an enteric coating solution is sprayed onto the core. Suitable solvents used in the enteric coating process include alcohols such as ethanol, ketones such as acetone, and halogenated hydrocarbon solvents such as dichloromethane (CH₂Cl₂), and a mixed solvent of these solvents may be used. A softening agent such as di-n-butylphthalate or triacetin is added to the coating solution at a ratio of 1 to about 0.05-0.3 (coating material to softening agent). It is appropriate to continuously perform the spraying process, and it is possible to adjust the spraying amount in consideration of the coating conditions. The spraying pressure may be variously adjusted, and satisfactory results are typically obtained at a spraying pressure of about 1-1.5 bar.

As used herein, the term 'prevention' is associated with averting, delaying, impeding, or hindering progression of a disease in order to reduce onset of the same.

As used herein, the term 'treatment' is associated with caring for a subject suffering from a disease in order to ameliorate, cure, or reduce the symptoms of the disease or to reduce or arrest the progression of the disease.

Another aspect of the present invention pertains to a functional food composition for use in the prevention or amelioration of climacteric symptoms containing *Lactobacillus gasseri* BNR17 deposited as KCTC 10902BP as an active ingredient.

The prevention or amelioration of climacteric symptoms may be selected from the group consisting of (i) prevention or amelioration of osteoporosis, (ii) prevention or alleviation of involutional depression, (iii) prevention or amelioration of pain hypersensitivity, and (iv) prevention or amelioration of vaginal dryness, but the present invention is not limited thereto.

In the present invention, the functional food composition for use may further include a *Sophora japonica* extract, but is not limited thereto, and may be administered in combination with a known material having proven efficacy in preventing or ameliorating climacteric symptoms in order to enhance the effect thereof.

In the present invention, the *Sophora japonica* extract may be a *Sophora japonica* fruit extract, but is not limited thereto.

The *Sophora japonica* extract may be administered in a daily dosage of 0.1 to 1000 mg, preferably 1 to 500 mg, more preferably 30 to 300 mg, but the present invention is not limited thereto.

The functional food composition disclosed herein includes a composition that is added to food, and may be easily utilized as a food, for example, a main material of food, a supplementary material of food, a food additive, a functional health food, or a functional beverage, in order to exert effects such as amelioration, alleviation, and prevention of climacteric symptoms, but the present invention is not limited thereto.

The food is a natural product or processed product containing one or more nutrients, preferably a product that is directly converted into an edible form through certain processing, usually encompassing all of foods, food additives, functional health foods, and functional beverages.

Examples of the food to which the functional food composition for use according to the present invention may be added include various foods, beverages, gums, teas, vitamin complexes, functional foods, and the like. Additionally, examples of food include, but are not limited to, special nutritional foods (e.g. modified milk, infant/toddler food, etc.), processed meat products, fish products, soybean curd, jellied food products, noodles (e.g. ramen, noodles, etc.), breads, health supplements, seasonings (e.g. soy sauce, soybean paste, red pepper paste, mixed paste, etc.), sauces, confectioneries (e.g. snacks), candy, chocolate, gum, ice cream, dairy products (e.g. fermented milk, cheese, etc.), other processed foods, kimchi, pickled foods (various types of kimchi, pickles, etc.), beverages (e.g. fruit drinks, vegetable drinks, soy milk, fermented drinks, etc.), natural seasonings (e.g. ramen soup, etc.), and the like. The food, beverage, or food additive may be prepared through typical methods.

Functional health foods are a group of foods to which value is added so that the function thereof is exerted and exhibited for a predetermined purpose using physical, biochemical, or bioengineering techniques, or a processed food designed so that the control functions of the food composition, such as regulation of biological defense rhythm, prevention of disease, and recovery from disease are sufficiently exhibited *in vivo.* The functional food may include a food supplement that is culinarily acceptable, and may further include an appropriate carrier, excipient, or diluent commonly used in the manufacture of functional food.

As used herein, "functional beverage" is a generic term for drink products consumed to quench thirst or to enjoy the taste thereof. There is no particular limitation on other ingredients, so long as the composition, which is the essential ingredient, is included at the indicated ratio, and various flavoring agents or natural carbohydrates may be included as additional ingredients, like typical beverages.

Furthermore, in addition to those described above, the food containing the functional food composition for use of the present invention may include various nutrients, vitamins, minerals (electrolytes), flavoring agents such as synthetic and natural flavoring agents, coloring agents, fillers (cheese, chocolate, etc.), pectic acids and salts thereof, alginic acid and salts thereof, organic acids, protective colloidal thickeners, pH adjusters, stabilizers, preservatives, glycerin, alcohols, carbonation agents used in carbonated beverages, etc., used alone or in combination.

In the food containing the functional food composition for use of the present invention, the composition for use according to the present invention may be included in an amount of 0.001 wt% to 100 wt%, preferably 1 wt% to 99 wt%, based on the total weight of the food. The composition may be included in an amount of 1 wt% to 80 wt%, or 1 to 90 wt%. The composition may be included in the beverage in an amount of 0.001 g to 10 g, preferably 0.01 g to 1 g, per 100 ml. Upon long-term intake for health and hygiene purposes or for health maintenance purposes, the amount thereof may be equal to or less than the above lower limit, and since the active ingredient has no problem in terms of safety, the composition may be used in an amount equal to or greater than the above upper limit, so the amount of the composition is not limited to the above range.

The functional food composition for use of the present invention may include only the BNR 17 strain and optionally the *Sophora japonica* extract, but may be added to an acceptable carrier or prepared in the form of a composition suitable for ingestion by a human or animal. Specifically, it may be added to food that does not contain other probiotic bacteria and food that already contains some probiotic bacteria. Other microorganisms that may be used together with the strain of the present invention are those suitable for ingestion by humans or animals and having probiotic activity capable of inhibiting the proliferation of pathogenic bacteria or improving the microbial balance in the mammalian intestinal tract when ingested, and are not particularly limited. Examples of such probiotic microorganisms include yeast including *Saccharomyces, Candida, Pichia,* and *Torulopsis,* fungi such as *Aspergillus, Rhizopus, Mucor, Penicillium,* etc., and bacteria belonging to the genera *Lactobacillus, Bifidobacterium, Leuconostoc, Lactococcus, Bacillus, Streptococcus, Propionibacterium, Enterococcus,* and *Pediococcus.* Specific examples of suitable probiotic microorganisms include *Saccharomyces cerevisiae, Bacillus coagulans, Bacillus licheniformis, Bacillus subtilis, Bifidobacterium bifidum, Bifidobacterium infantis, Bifidobacterium longum, Enterococcus faecium, Enterococcus faecalis, Lactobacillus acidophilus, Lactobacillus alimentarius, Lactobacillus casei, Lactobacillus curvatus, Lactobacillus delbrueckii, Lactobacillus johnsonii, Lactobacillus farciminis, Lactobacillus gasseri, Lactobacillus helveticus, Lactobacillus rhamnosus, Lactobacillus reuteri, Lactobacillus sakei, Lactococcus lactis, Pediococcus acidilactici,* etc. Preferably, the functional food composition for use of the present invention further includes a mixture of probiotic microorganisms having excellent probiotic activity and immune activity enhancement, so the effect thereof is more enhanced. Examples of carriers that may be used in the functional food composition for use of the present invention include extenders, high-fiber additives, encapsulating agents, lipids, freeze-drying protectants, and the like, and such carriers are well known in the art. The composition for use of the present invention may be in lyophilized or encapsulated form, or in the form of a culture suspension or dry powder. The amount of the carrier may be 0.001 to 90 wt%, 0.01 to 50 wt%, or 0.1 to 20 wt%, such as 0.001 to 1 wt%, but is not limited thereto.

The BNR17 strain of the present invention has excellent acid resistance, bile resistance, and antibacterial activity, so it may be used as a seed to produce milk and various other products fermented using lactic acid bacteria. Here, fermented milk foods include yogurt, calpis, cheese, butter, etc. and fermented products include soybean curd, soybean paste, fast-fermented soybean paste, jelly, kimchi, and the like, but is not necessarily limited thereto. The fermented milk or other fermented product may be easily obtained merely by replacing the strain with the lactic acid bacterial strain in a typical preparation method.

As described above, the composition for use of the present invention may be a food or a food additive, and the food is preferably a fermented food or a functional health food. In addition, the composition for use of the present invention may be in powder or granular form, and the powder or granular form may be easily prepared through a typical method known in the art.

The pharmaceutical composition for use according to the present invention is capable of exhibiting at least one effect of treating climacteric syndrome selected from the group consisting of (i) amelioration of osteoporosis, (ii) alleviation of involutional depression, (iii) amelioration of pain hypersensitivity, and (iv) amelioration of vaginal dryness, not only in a subject having normal body weight consuming a regular diet, but also in an obese subject consuming a high-sucrose diet.

In addition, the functional food composition for use is capable of exhibiting at least one effect of preventing or ameliorating climacteric symptoms selected from the group consisting of (i) prevention or amelioration of osteoporosis, (ii) prevention or alleviation of involutional depression, (iii) prevention or amelioration of pain hypersensitivity, and (iv) prevention or amelioration of vaginal dryness, not only in a subject having normal body weight consuming a regular diet, but also in an obese subject consuming a high-sucrose diet.

Disclosed herein, but not part of the claimed invention, is a method of treating climacteric syndrome including administering *Lactobacillus gasseri* BNR17 to a subject requiring treatment for climacteric syndrome.

Disclosed herein, but not part of the claimed invention, is a method of preventing or ameliorating climacteric symptoms including administering *Lactobacillus gasseri* BNR17 to a subject requiring prevention or amelioration of climacteric symptoms.

Disclosed herein, but not part of the claimed invention, is the use of *Lactobacillus gasseri* BNR17 for the treatment of climacteric syndrome or the prevention or amelioration of climacteric symptoms.

Disclosed herein, but not part of the claimed invention, is the use of *Lactobacillus gasseri* BNR17 for the manufacture of a medicament for the treatment of climacteric syndrome.

Disclosed herein, but not part of the claimed invention, is the use of *Lactobacillus gasseri* BNR17 for the manufacture of a functional food for the prevention or amelioration of climacteric symptoms.

A better understanding of the present invention and disclosure may be obtained through the following examples. The scope of the present invention is as defined by the appended claims.

### Examples

### Example 1. Experimental method and preparation

### 1-1. Construction of climacteric experimental animal model and sample administration

As experimental animals, 9-week-old female rats (Sprague-Dawley), subjected to ovariectomy and treatment at 7 weeks of age, were purchased from Daehan Biolink (DBL Co., Ltd), acclimatized for 1 week, and then randomly grouped and used. Experimental animals were bred by placing two animals per cage in a breeding chamber (JD-SY-02DS-C, Jungdo B&P, Korea) in which temperature and humidity were maintained automatically. The breeding chamber was maintained under conditions of a temperature of 23±1°C, a humidity of 55±5%, lighting for 12 hours, and light intensity of 150-300 Lux. In the experimental groups, normal solid feed (TEKLAD CERTIFIED IRRADIATED GLOBAL 18% PROTEIN RODENT DIET 2918C, Harlan TEKLAD, USA) and high-sugar solid feed (AIN-76A Purified Diet, Envigo, USA) were freely provided. The breeding boxes, feeders, and water bottles were exchanged twice a week, and feed, litter materials, and water bottles used for animal breeding were sterilized before use. Animal breeding was carried out according to the standard operating guidelines of Sirnagen Therapeutics.

A sample was administered to each of the experimental groups in the dosage shown in Table 1 below, and the experimental animals were divided into a total of 4 groups from G1 to G4, including a normal experimental group (G1), a negative control group (G2), a sample administration group (G3), and a positive control group (G4). In preparing the administration sample, *Lactobacillus gasseri* BNR17 lactic acid bacteria are in a dry powder form for oral administration and contain a freeze-drying protectant, so the freeze-drying protectant (10% skim milk + l% soluble starch) was administered in the same amount to G2 (the negative control group), G3 (the sample administration group), and G4 (the positive control group), but not to G1 (the normal experimental group). The *Sophora japonica* fruit extract used in the positive control group was a powder sample processed through an extraction method using water and ethanol, and was purchased from BTC and used.

**[Table 1]**

| Group | | Sample dosage and administration method (twice a day, orally) |
|---|---|---|
| G1 (Normal experimental group, non-ovariectomized group), n = 6 | | PBS |
| Ovariectomized group (OVX) + High-sucrose diet (HSD) | G2 (Negative control group), n = 8 | PBS |
| | G3 *(Lactobacillus gasseri* BNR17, KCTC 10902 BP), n = 8 | 1 X 10¹⁰ CFU |
| | G4 (Positive control group, *Sophora japonica* fruit extract), n = 8 | 100 mg |

The test material was weighed daily using an electronic scale (Adventurer Pro AVG114C, Ohaus, USA), diluted to an appropriate volume with PBS (C-9024, Bioneer, Korea), and administered orally twice daily for 14 weeks using a sonde.

### 1-2. Autopsy and sampling

The experimental animals were fasted starting 16 hours before autopsy from the end of the experiment, and body weight before autopsy was measured using an electronic scale (FX-2000i, Korea AND, Korea). The animals were anesthetized through exposure to a respiratory anesthetic (Terrel^{™} Isoflurane, Piramal, U.S.A.) using a respiratory anesthesia device (Rodent Circuit Controller; RC2, VETEQUIP^{®}, USA). After complete anesthesia, the abdominal aorta was exposed through laparotomy. Blood was collected from the abdominal aorta using a 10 ml disposable syringe (Korea Vaccine, Korea), after which 500 µl thereof was placed in an EDTA tube (367841, BD, USA) and the remaining amount was stored in an SST tube (367955, BD, USA). Whole blood was thoroughly mixed with an anticoagulant and stored in a refrigerator. The whole blood placed in the SST tube was allowed to stand at room temperature for 30 minutes, followed by centrifugation at 4°C at 3500 RPM for 15 minutes using a centrifuge (Centrifuge Combi 514R, Hanil Scientific Inc., Korea), after which the supernatant was rapidly cooled in liquid nitrogen and stored at -70°C (Deep Freezer, NF-400SF, Nihon Freezer Co., Japan) until analysis.

The animals were fasted for 16 hours on the last day of the experiment and then placed on a fasting net to obtain urine in an amount of 0.5-1 ml. After centrifugation at 4°C at 8000 RPM for 15 minutes using a centrifuge, the supernatant from which the precipitate was removed was collected, and then stored at -70°C until analysis. Osteocalcin (CSB-E05129r, CUSABIO, China), calcitonin (CSB-E05132r, CUSABIO, China), and 5-HT-2A (CSB-E14956r, CUSABIO, China), for analysis of climacteric indicators in the serum, and deoxypyridinoline (CSB-E08400r, CUSABIO, China), for analysis of a climacteric indicator in the urine, were purchased and used for analysis using a microplate reader (Infinity M200 pro, TECAN, Switzerland) through an ELISA kit method.

### 1-3. Analysis of changes in biochemical and climacteric indicators in blood and urine

In order to measure changes in biochemical indicators in the blood after menopause, blood was collected according to the method of Example 1-2 at the end of the experiment, and the concentrations of alanine aminotransferase (ALT), aspartate aminotransferase (AST), blood urea nitrogen (BUN), calcitonin, osteocalcin, 5-HT-2A (serotonin), and deoxypyridinoline were measured in the blood test. High levels of ALT and AST in basic liver function tests may indicate the development of fatty liver and various types of hepatitis. BUN is an indicator capable of confirming kidney function by measuring the content of nitrogen having the form of urea in the blood. Calcitonin, which is a calcium regulatory hormone, causes problems during bone reproduction when insufficient, including bone weakness, osteoporosis, and pain, and the concentration thereof is lowered in climacteric patients and thus a prescription is given to supplement the same. Osteocalcin, which is a known bone formation indicator, is used as a single indicator reflecting the extent of bone formation, and is increased in bone diseases. 5-HT-2A (serotonin) is the neurotransmitter, and the amount thereof decreases after menopause, and this decrease causes involutional depression. Since deoxypyridinoline exists only in bone and dentin, it is known to have relatively high specificity in reflecting bone metabolism. When bone is resorbed, deoxypyridinoline is not metabolized in the body but is excreted in the urine, so it is a useful indicator for evaluation of bone resorption.

### 1-4. Analysis of pain sensitivity (von Frey filament test)

According to previous reports, it is known that sensitivity to pain increases after ovariectomy. In experimental animal models, the results thereof were evaluated by quantifying the extent of mechanical allodynia, and a von Frey filament test method was employed. The experimental animals were placed in a box having a wire mesh test table installed therein and allowed to acclimatize for 15 minutes, and the mechanical withdrawal threshold (g) was determined using a von Frey filament (JD-SI-11F, Jungdo B&P, Korea). The filament was brought into perpendicular contact with the center of the rear right foot and held there for 5-6 seconds. If the experimental animal showed a quick avoidance response, flinched immediately while removing the foot from the filament, or licked the sole of the foot, it was considered a positive response. By sequentially proceeding from weak filament stimulation to strong filament stimulation, the minimum stimulation magnitude showing a positive response was determined to be a threshold.

### 1-5. Test for change in cornified vaginal epithelial cells

Since vaginal dryness is one of the most representative climacteric symptoms, in this experiment, it was confirmed whether the test material could help climacteric symptoms by observing changes in vaginal epithelial cells in each group. The vaginal cornification test is an important indicator for observing changes in the urinary system as a biomarker of animal tests to confirm health functionalities of climacteric women among the functional evaluation guidelines for functional health foods. Before autopsy, vaginal mucosal cells were removed through scraping, spread on a slide, and fixed with formalin (GD4018, GD Chem, Korea) for 3 minutes, after which remaining formalin was removed, staining with a methylene blue solution (03978-250ML, Sigma, USA) for 5 minutes was performed, and 3-4 sites were randomly observed at a 100X magnification using a microscope (235095(Ts2-FL), Nikon, Japan) to determine the proportion of cornified epithelial cells among vaginal mucosal cells. A high proportion of cornified epithelial cells among vaginal mucosal cells was interpreted to mean that the level of vaginal cornification decreased and that the vaginal mucosa was made flexible.

### 1-6. Measurement of femoral bone density

The animals were sacrificed at the end of the experiment according to the method of Example 1-2, the femoral head of each animal was carefully separated, the skin tissue and muscle tissue were removed therefrom, and the femoral head was fixed and stored in formalin (GD4018, GD Chem, Korea). The bone density of the femur was measured using Micro-CT equipment (Quntum GX, PerkinElmer, USA).

### Example 2. Analysis of climacteric animal model through ovariectomy

### 2-1. Analysis of biochemical indicators in blood

In order to measure changes in biochemical indicators in the blood after ovariectomy, the animals were sacrificed at the end of the experiment, and blood was then collected therefrom and analyzed according to the methods of Examples 1-2 and 1-3.

Consequently, as shown in Table 2 below, both ALT and AST levels in the blood were increased in G2 (the negative control group) and G4 (the positive control group) compared to G1 (the normal experimental group), but were decreased in the G3 group (the group administered with BNR17) to approximately the same levels as G1 (the normal experimental group). BUN showed no significant difference across groups. Therefore, when taking BNR17 for 14 weeks, it was confirmed that there were no adverse effects on liver or kidney function and also that a beneficial effect was exhibited on liver function abnormality in the ovariectomized group.

**[Table 2]**

| Group | | | ALT | AST | BUN |
|---|---|---|---|---|---|
| G1 | Normal experimental group | | 31.7±4 | 108.2±13.1 | 14.5±0.7 |
| G2 | OVX + HSD | Negative control group (PBS) | 51.9±7.1 | 223.9±35 | 12.6±0.6 |
| G3 | | BNR17 | 36.2±4.2 | 156.1±34 | 12.5±0.6 |
| G4 | | Positive control group *(Sophora japonica* fruit extract) | 64±18.5 | 272.3±54.3 | 12.1±1 |

### 2-2. Analysis of climacteric indicators in blood

The animals were sacrificed at the end of the experiment, blood was collected according to the methods of Examples 1-2 and 1-3, and climacteric indicators in the blood were analyzed.

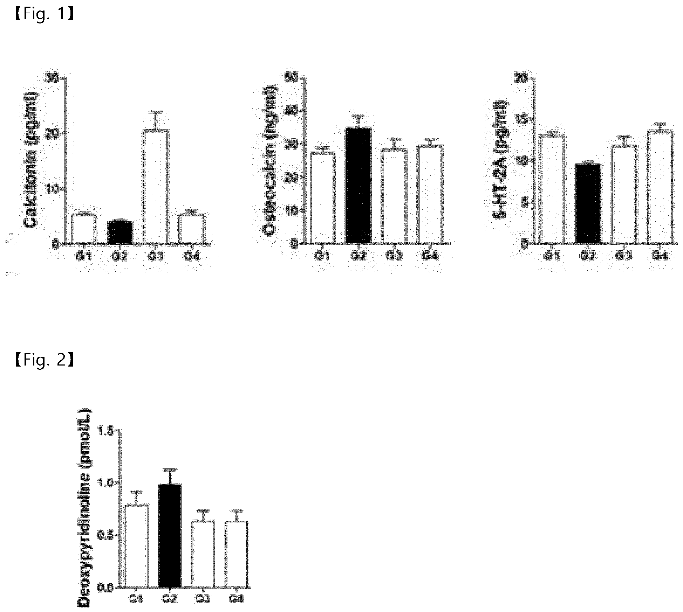

Consequently, as shown in FIG. 1, it was confirmed that the amount of calcitonin in the blood was significantly increased in the G3 group (the group administered with BNR17) compared to the G2 group (the negative control group) and the G4 group (the positive control group) (G2: 4.03±0.29 pg/ml; G3: 20.51±3.32 pg/ml; G4: 5.31±0.67 pg/ml).

Also, it was confirmed that osteocalcin, which was increased in G2 (the negative control group) compared to G1 (the normal experimental group), was restored to the levels of G1 (the normal experimental group) and G4 (the positive control group) in the G3 group (the group administered with BNR17) (G2: 34.84±3.89 ng/ml; G3: 28.5±3.25 ng/ml; G4: 24.51±4.04 ng/ml) .

5-HT-2A (serotonin), which was confirmed to be decreased in the G2 group (the negative control group) compared to the G1 group (the normal experimental group), was also restored to the levels of G1 (the normal experimental group) and G4 (the positive control group) in the G3 group (the group administered with BNR17) (G2: 9.57±0.32 pg/ml; G3: 11.74±1.11 pg/ml; G4: 13.47±0.98 pg/ml) .

These results showed that the blood indicators associated with osteoporosis occurring during climacterium were the same as or better than in the group administered with the *Sophora japonica* fruit extract, which is the positive control group, due to administration of BNR17, indicating that BNR17 according to the present invention is effective at alleviating involutional depression.

### 2-3. Analysis of climacteric indicator in urine

The animals were sacrificed at the end of the experiment, urine was collected therefrom according to the methods of Examples 1-2 and 1-3, and the climacteric indicator in the urine was analyzed.

Consequently, as shown in FIG. 2, the amount of deoxypyridinoline in the blood was 0.63±0.1 pmol/L in the G3 group (the group administered with BNR17), which was regarded as significantly decreased compared to 0.98±0.14 pmol/L for the G2 group (the negative control group). This value was lower than 0.79±0.13 pmol/L, which was the level for G1 (the normal experimental group) .

Based on the above results, it was confirmed that BNR17 administration is capable of ameliorating osteoporosis that may occur during climacterium.

### 2-4. Analysis of pain sensitivity

In order to compare changes in pain sensitivity due to sample administration in a climacteric animal model, pain sensitivity was measured by performing the von Frey filament test according to the method of Example 1-4.

Consequently, as shown in Table 3 below, in the G2 group (the negative control group) subjected to ovariectomy, the threshold value of the force at which pain was felt was significantly lowered compared to the G1 group (the normal experimental group), indicating that pain sensitivity increased. However, in the G3 group (the group administered with BNR17) to which BNR17 was administered after ovariectomy, the threshold value was increased to a level equal to or higher than the level for G1 (the normal experimental group), and this value was determined to be superior to that observed for the G4 positive control group (the group administered with *Sophora japonica* fruit extract).

Based on the above results, it was confirmed that BNR17 administration is capable of ameliorating increased pain sensitivity, which is a climacteric symptom.

**[Table 3]**

| Group | | | Mechanical withdrawal threshold |
|---|---|---|---|
| G1 | Normal experimental group | | 18±7.6 |
| G2 | OVX + HSD | Negative control group (PBS) | 6.26±2.55 |
| G3 | | BNR17 | 19.63±8.01 |
| G4 | | Positive control group *(Sophora japonica* fruit extract) | 15.7±6.4 |

### 2-5. Analysis of cornified vaginal epithelial cells

In order to compare changes in vaginal dryness due to sample administration in a climacteric animal model, a vaginal cornification experiment was performed according to the method of Example 1-5.

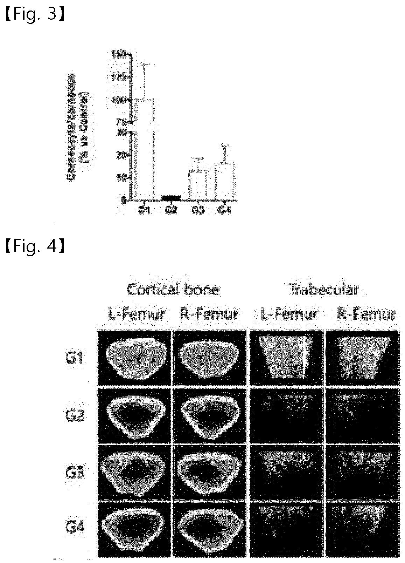

Consequently, as shown in FIG. 3, through comparison of distribution of cornified cells, the G2 group (the negative control group) showed a marked reduction in distribution of cornified epithelial cells (G1: 100±38.87%; G2: 1.83±0.39%) compared to the G1 group (the normal experimental group), but the G3 group (the group administered with BNR17) exhibited increased distribution of cornified epithelial cells to a level similar to that for the G4 positive control group (the group administered with *Sophora japonica* fruit extract) (G3: 12.85±5.63 %; G4: 16.16±7.73 %).

Based on the above results, it was found that BNR17 administration is effective at alleviating vaginal dryness by reducing the level of vaginal cornification and making the vaginal mucosa flexible.

### 2-6. Analysis of femoral bone density

Bone mineral density (BMD) was measured using a bone density measurement system (Quantum GX, PerkinElmer, USA) through the method of Example 1-6.

Consequently, as shown in FIG. 4, bone density was observed to be significantly decreased in the G2 group (the negative control group) compared to the G1 group (the normal experimental group), but was considerably increased in the G3 group (the group administered with BNR17) compared to the G2 group (the negative control group), and the effect in the G3 group was equal to or greater than that in the G4 group (the group administered with *Sophora japonica* fruit extract).

Based on the above results, it was confirmed that BNR17 is effective at improving bone density.

Name of depository institution: Korea Research Institute of Bioscience and Biotechnology
Accession number: KCTC10902BP
Date of deposit: 20060123

### [Industrial Applicability]

According to the present invention, *Lactobacillus gasseri* BNR17 is a breast milk lactic acid bacterium, the safety of which has been proven by the Ministry of Food and Drug Safety. When the lactic acid bacterium is administered, various climacteric symptoms such as osteoporosis, involutional depression, pain hypersensitivity, and vaginal dryness can be effectively alleviated. *Lactobacillus gasseri* BNR17 can be efficiently used in a pharmaceutical or food composition for treating, preventing, or ameliorating climacteric syndrome or climacteric symptoms.

## Claims

1. A pharmaceutical composition for use in the treatment of climacteric syndrome comprising *Lactobacillus gasseri* BNR17 deposited as KCTC 10902BP as an active ingredient.

2. The pharmaceutical composition for use according to claim 1, wherein the treatment of climacteric syndrome is selected from the group consisting of:
(i) amelioration of osteoporosis;
(ii) alleviation of involutional depression;
(iii) amelioration of pain hypersensitivity; and
(iv) amelioration of vaginal dryness.

3. The pharmaceutical composition for use according to claim 1, further comprising a *Sophora japonica* extract.

4. The pharmaceutical composition for use according to claim 3, wherein the *Sophora japonica* extract is a *Sophora japonica* fruit extract.

5. A functional food composition for use in the prevention or amelioration of climacteric symptoms comprising *Lactobacillus gasseri* BNR17 deposited as KCTC 10902BP as an active ingredient.

6. The functional food composition for use according to claim 5, wherein the prevention or amelioration of climacteric symptoms are selected from the group consisting of:
(i) amelioration of osteoporosis;
(ii) alleviation of involutional depression;
(iii) amelioration of pain hypersensitivity; and
(iv) amelioration of vaginal dryness.

7. The functional food composition for use according to claim 5, further comprising a *Sophora japonica* extract.

8. The functional food composition for use according to claim 7, wherein the *Sophora japonica* extract is a *Sophora japonica* fruit extract.

## Patentansprüche

1. Pharmazeutische Zusammensetzung zur Verwendung bei der Behandlung des klimakterischen Syndroms, die Lactobacillus gasseri BNR17, das als KCTC 10902BP hinterlegt ist, als Wirkbestandteil umfasst.

2. Pharmazeutische Zusammensetzung zur Verwendung nach Anspruch 1, wobei die Behandlung des klimakterischen Syndroms aus folgender Gruppe ausgewählt ist:
(i) Linderung von Osteoporose;
(ii) Linderung von Involutionsdepression;
(iii) Linderung von Schmerzüberempfindlichkeit; und
(iv) Linderung von Vaginaltrockenheit.

3. Pharmazeutische Zusammensetzung zur Verwendung nach Anspruch 1, die weiters einen Sophora-japonica-Extrakt umfasst.

4. Pharmazeutische Zusammensetzung zur Verwendung nach Anspruch 3, wobei der Sophora-japonica-Extrakt ein Sophora-japonica-Fruchtextrakt ist.

5. Funktionelle Lebensmittelzusammensetzung zur Verwendung bei der Vorbeugung oder Linderung von klimakterischen Symptomen, die Lactobacillus gasseri BNR17, das als KCTC 10902BP hinterlegt ist, als Wirkbestandteil umfasst.

6. Funktionelle Lebensmittelzusammensetzung zur Verwendung nach Anspruch 5, wobei die Vorbeugung oder Linderung von klimakterischen Symptomen aus folgender Gruppe ausgewählt ist:
(i) Linderung von Osteoporose;
(ii) Linderung von Involutionsdepression;
(iii) Linderung von Schmerzüberempfindlichkeit; und
(iv) Linderung von Vaginaltrockenheit.

7. Funktionelle Lebensmittelzusammensetzung zur Verwendung nach Anspruch 5, die weiters einen Sophora-japonica-Extrakt umfasst.

8. Funktionelle Lebensmittelzusammensetzung zur Verwendung nach Anspruch 7, wobei der Sophora-japonica-Extrakt ein Sophora-japonica-Fruchtextrakt ist.

## Revendications

1. Composition pharmaceutique pour utilisation dans le traitement du syndrome climatérique comprenant du *Lactobacillus gasseri* BNR17 déposé sous le numéro KCTC 10902BP en tant que principe actif.

2. Composition pharmaceutique pour utilisation selon la revendication 1, dans laquelle le traitement du syndrome climatérique est choisi parmi le groupe comprenant :
(i) l'amélioration de l'ostéoporose ;
(ii) le soulagement de la dépression involutionnelle ;
(iii) l'amélioration de l'hypersensibilité à la douleur ; et
(iv) l'amélioration de la sécheresse vaginale.

3. Composition pharmaceutique pour utilisation selon la revendication 1, comprenant en outre un extrait de *Sophora japonica.*

4. Composition pharmaceutique pour utilisation selon la revendication 3, dans laquelle l'extrait de *Sophora japonica* est un extrait de fruit de *Sophora japonica.*

5. Composition alimentaire fonctionnelle pour utilisation dans la prévention ou l'amélioration de symptômes climatériques comprenant du *Lactobacillus gasseri* BNR17 déposé sous le numéro KCTC 10902BP en tant que principe actif.

6. Composition alimentaire fonctionnelle pour utilisation selon la revendication 5, dans laquelle la prévention ou l'amélioration de symptômes climatériques sont choisies dans le groupe comprenant :
(i) l'amélioration de l'ostéoporose ;
(ii) le soulagement de la dépression involutionnelle ;
(iii) l'amélioration de l'hypersensibilité à la douleur ; et
(iv) l'amélioration de la sécheresse vaginale.

7. Composition alimentaire fonctionnelle pour utilisation selon la revendication 5, comprenant en outre un extrait de *Sophora japonica.*

8. Composition alimentaire fonctionnelle pour utilisation selon la revendication 7, dans laquelle l'extrait de *Sophora japonica* est un extrait de fruit de *Sophora japonica.*
